Europäisches Patentamt

European Patent Office

Office européen des brevets

(11) Numéro de publication: **0 362 093**

**A2**

(12) **DEMANDE DE BREVET EUROPEEN**

(21) Numéro de dépôt: 89440065.4

(51) Int. Cl.⁵: **A61N 1/39**

(22) Date de dépôt: 06.07.89

(30) Priorité: 06.07.88 FR 8809377

(43) Date de publication de la demande:
04.04.90 Bulletin 90/14

(84) Etats contractants désignés:
AT BE CH DE ES GB GR IT LI LU NL SE

(71) Demandeur: **ATESYS, société anonyme**
**44, rue de l'Industrie**
**F-67160 Wissembourg(FR)**

(72) Inventeur: **Cansell, Albert**
**7, rue du Berger Altenstadt**
**F-67160 Wissembourg(FR)**
Inventeur: **Foeller, Clément**
**8, rue du Maréchal Mac-Mahon**
**F-67110 Reichsoffen(FR)**

(74) Mandataire: **Metz, Paul**
**Cabinet METZ PATNI 63, rue de la Ganzau**
**B.P. 63**
**F-67024 Strasbourg Cédex(FR)**

(54) **Circuit de charge-décharge pour le condensateur d'un défibrillateur cardiaque.**

(57) Circuit de charge-décharge pour le condensateur d'un défibrillateur cardiaque.

Circuit caractérisé en ce que le circuit de décharge comporte outre le condensateur C, au moins un éclateur E en série avec le condensateur C, éclateur couplé optiquement à un élément optoélectronique relié à une entrée (5) de remise à zéro RAZ d'un circuit de commande et d'inhibition CCI destiné à arrêter le fonctionnement du circuit de charge (2) pour empêcher toute recharge automatique du condensateur C après une première décharge.

Cette invention intéresse les constructeurs de défibrillateurs et, plus généralement, les professionnels de l'électronique médicale.

FIG.1

## Circuit de charge-décharge pour le condensateur d'un défibrillateur cardiaque.

La présente invention se rapporte à un circuit de charge-décharge du condensateur pour un défibrillateur cardiaque implantable.

Un défibrillateur cardiaque implantable est généralement constitué d'un condensateur électrique que l'on charge à un niveau donné de haute tension, et que l'on décharge à travers le coeur d'un patient au moyen d'au moins deux électrodes, le plus souvent implantées sur la paroi extérieure du coeur appelée épicarde.

Dans certains cas, on peut aussi utiliser des électrodes placées à l'intérieur du coeur ou sur le thorax en position sous-cutanée.

Le choc de défibrillation doit être délivré automatiquement dès que l'appareil détecte un trouble grave du rythme (fibrillation ventriculaire ou tachycardie ventriculaire). Cette détection s'effectue habituellement en surveillant les signaux électrophysiologiques que l'on recueille sur le coeur. Elle commande avec l'aide d'un circuit décisionnel le processus qui aboutira automatiquement à la décharge de défibrillation.

Les circuits de décharge connus utilisables pour un tel défibrillateur automatique implantable comportent un élément interrupteur commandé, destiné à fermer le circuit de décharge dès l'application du signal de commande.

Les éléments connus actuellement et pouvant convenir sont les suivants :

. composants semi-conducteurs (transistors, thyristors etc...) dont le principal inconvénient concerne la difficulté de les réaliser sous un volume réduit pour des tensions élevées (1000 à 3000 volts) comme l'exigent, dans certains cas, les impératifs d'environnement de ce circuit;

. les relais électro-mécaniques qui présentent la même impossibilité de miniaturisation que les précédents ;

. les tubes à décharge commandés qui , avantageux de par leur taille, nécessitent cependant une très haute tension de commande sur leur grille (environ 20 KV), ce qui est difficile à obtenir dans le faible volume de l'appareil.

La présente invention a pour objectif de réaliser un circuit de charge-décharge pour défibrillateur implantable qui d'une part n'ait pas les inconvénients décrits ci-dessus et d'autre part permette d'obtenir une décharge entièrement automatique à maîtrise totale de la recharge.

Elle est basée sur l'utilisation dans le circuit de décharge d'un tube à décharge (éclateur à gaz rare, lampe à néon, etc...), non commandé, placé en série entre une armature du condensateur et une des deux électrodes placées sur le coeur.

Une telle solution, si elle s'avère satisfaisante pour obtenir une décharge automatique, s'avère totalement insuffisante pour maîtriser la recharge.

Le problème posé pour un tel dispositif est que, lorsque le condensateur est déchargé, le tube à décharge se désamorce et redevient isolant, le condensateur se recharge une nouvelle fois jusqu'à ce que la tension d'amorçage de l'éclateur provoque une nouvelle décharge, et ainsi de suite.

Bien entendu, un tel régime correspondant à celui d'un oscillateur ne convient pas du tout à une application de défibrillation dans laquelle il s'avère hautement important de rester maître de chaque décision de décharge, c'est-à-dire d'obtenir une décharge à déclenchement commandé.

On veut ainsi pouvoir commander à chaque fois une charge-décharge unique, par un moyen de commande extérieur au circuit de décharge et sur décision résultant d'un programme intérieur à l'appareil, en fonction des signaux cardiaques mesurés.

Le but de la présente invention consiste, en utilisant un tel éclateur non commandé, à verrouiller à chaque décharge, la recharge du condensateur et plus particulièrement à verrouiller la commande de son circuit de charge après chaque décharge, de manière à rendre toute charge ultérieure conditionnelle d'une nouvelle décision et action de commande.

Pour ce faire, on se propose d'arrêter le générateur haute-tension de charge dès l'instant où la décharge vient de se produire, et ce jusqu'à l'arrivée d'une nouvelle décision de provoquer une nouvelle décharge de défibrillation.

Ce but est atteint selon l'invention par un circuit de charge-décharge du condensateur d'un défibrillateur implantable présentant une source de courant continu, un circuit de haute tension de charge, un condensateur, au moins un éclateur en série avec le condensateur, et les électrodes appliquées au patient, l'éclateur étant coupé optiquement à un détecteur optoélectronique relié à l'entrée de verrouillage d'un circuit de commande et d'inhibition du fonctionnement du circuit de charge destiné à commander la charge puis à empêcher toute recharge automatique du condensateur après une première décharge.

De nombreux avantages découlent de l'existence et de l'utilisation d'un tel circuit :

. possibilité de garder un degré de miniaturisation avancé

. grande fiabilité de fonctionnement

. faible coût

. simplicité des circuits d'exploitation

. sécurité de la décharge et de l'absence de recharge ultérieure

. intégration parfaite dans le circuit électronique

. faible consommation d'énergie.

La présente invention sera bien comprise à la lecture de la description qui suit, effectuée à titre d'exemple non limitatif sur un mode d'exécution en réfé rence aux dessins accompagnants dans lesquels :

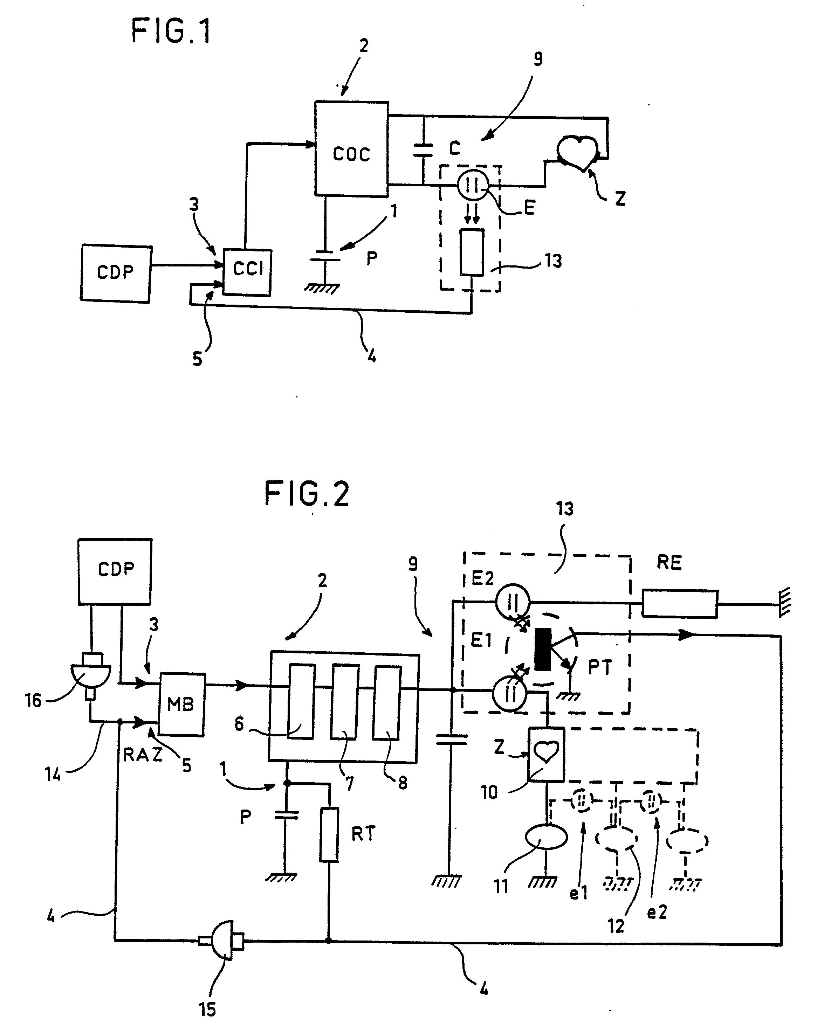

. la figure 1 est le schéma fonctionnel général de base illustrant le principe du circuit de charge-décharge pour défibrillateur cardiaque selon l'invention ;

. la figure 2 est le schéma du circuit de charge-décharge selon l'invention comprenant un phototransistor ainsi que deux éclateurs et plusieurs électrodes extérieures au coeur.

L'idée générale inventive consiste à prévoir dans le circuit de décharge du condensateur d'un défibrillateur cardiaque implanté un composant du type à amorçage c'est-à-dire devenant conducteur au-delà d'une certaine tension par un arc ou une décharge lumineuse et d'exploiter ce signal lumineux pour réaliser le verrouillage de la commande de fonctionnement du circuit de charge haute tension du condensateur.

On décrira tout d'abord dans leur généralité les ensembles fonctionnels du circuit de charge-décharge selon l'invention en référence à la figure 1.

Le circuit de charge-décharge selon l'invention se compose, de façon générale mais non limitative, des éléments suivants.

Une source de courant continu 1, par exemple une pile P au lithium-argent/oxyde de vanadium, alimente un circuit de charge 2 d'un condensateur C. Le circuit de charge 2 est commandé en fonctionnement et en arrêt par un circuit de commande et d'inhibition CCI, par exemple à changement(s) d'états à partir d'une part, d'un circuit décisionnel prioritaire CDP sur son entrée de commande 3 et d'autre part à partir d'une ligne de détection 4 sur son entrée d'inhibition 5.

Pour des raisons de facilité, le circuit de commande et d'inhibition CCI sera appelé simplement ci-après circuit de commande.

Le circuit de commande CCI est un circuit à un ou deux états stables, par exemple un circuit monostable M ou une bascule B.

Pour des raisons de clarté et de commodité, ce circuit de commande est référencé MB sur le schéma de la figure 2. Il présente l'entrée de commande 3 et l'entrée d'inhibition 5 qui est son entrée de réinitialisation ou RAZ (remise à zéro).

Une décision de déclencher une phase de charge-décharge se traduira, par exemple, par un niveau haut à la sortie du circuit MB qui a pour effet de mettre en fonctionnement le circuit de charge 2.

Malgré leur différence fonctionnelle, le circuit monostable M et la bascule B doivent être considérés comme des équivalents car ils provoquent les mêmes effets généraux à savoir la mise en fonctionnement du circuit de charge à chaque commande de déclenchement d'une telle phase.

Le circuit décisionnel prioritaire CDP commande la charge du condensateur C à partir des informations sur le fonctionnement du coeur détectées par ailleurs par des électrodes, notamment celles intérieures au coeur.

Pour des raisons supplémentaires de sécurité, si l'on utilise le circuit monostable M, les caractéristiques de ce circuit sont établies de façon qu'il présente une durée de maintien dans son état stable, légèrement supérieure à la durée de charge du condensateur C.

En effet si, pour une raison quelconque, le circuit de charge n'est pas coupé juste après la décharge, il le sera automatiquement à la fin de la période de maintien du monostable.

Le circuit de charge 2 se compose essentiellement d'un convertisseur continu/continu haute tension COC formé classiquement d'un oscillateur 6, d'un transformateur 7 et d'un redresseur 8.

Le circuit de charge 2 se referme sur un condensateur C commun au circuit de charge 2 et à un circuit de décharge 9, formé d'un éclateur E puis de l'impédance biologique Z du patient comprenant son coeur et les tissus séparant une électrode de décharge 10 intérieure au coeur d'une ou de plusieurs électrodes extérieures 11, 12 ou autres électrodes en contact avec ou extérieures au coeur.

A titre de sécurité, on prévoit un deuxième éclateur dont la tension d'amorçage est plus élevée. Les éclateurs sont alors référencés E1 et E2. L'éclateur E2 se referme sur une résistance RE. Il permet de pallier les défaillances du premier éclateur ou celles du circuit de décharge telles par exemple un défaut d'amorçage du premier éclateur E1 ou une rupture de fil allant vers les électrodes et de protéger le condensateur C contre une surcharge.

Le ou les éclateurs sont montés dans un boîtier optocoupleur 13 en liaison intime de couplage optique avec un détecteur photosensible, ou un récepteur optoélectronique, par exemple un phototransistor PT relié à la pile P par une résistance de charge RT. Le phototransistor PT reçoit le flux lumineux des éclateurs lors de la décharge du condensateur C et réinitialise automatiquement par la ligne 4 la bascule B ou le monostable M sur son entrée d'inhibition 5 de remise à zéro RAZ provoquant par exemple un niveau bas sur le circuit de commande, à l'entrée du circuit de charge 2. Ceci a pour effet de l'inhiber c'est-à-dire d'arrêter son fonctionnement, évitant ainsi la recharge automatique du condensateur C.

Le montage fonctionne en boucle fermée. Dès que la décision de provoquer une décharge de défibrillation est prise, le circuit décisionnel prioritaire CDP fait changer d'état le circuit monostable ou bistable MB mettant en fonctionnement le circuit de charge 2 qui charge le condensateur C.

Dès que la tension d'amorçage de l'éclateur E1 est atteinte, celui-ci devient conducteur et décharge automatiquement et complètement le condensateur C à travers le coeur.

L'éclair d'amorçage produit dans l'éclateur est capté par le récepteur photoélectrique PT qui fait changer d'état le circuit MB et coupe ainsi le fonctionnement du circuit de charge 2.

En cas d'anomalies diverses, par exemple si le choc de défibrillation n'est pas délivré ou si l'éclair n'est pas capté ou transmis, le convertisseur haute tension COC sera coupé automatiquement au bout d'une durée déterminée correspondant au temps de maintien du circuit monostable M constituant ainsi une sécurité supplémentaire contre un fonctionnement en régime permanent de charge-décharge.

Une décision d'annulation de la décharge est possible en intervenant, par une commande directe du circuit CDP sur l'entrée 5 de remise à zéro connue sous RAZ du circuit MB avant la charge complète du condensateur par une ligne annexe 14.

Les deux lignes 4 et 14 arrivant à l'entrée RAZ du circuit de commande et d'inhibition CCI permettant d'arrêter la charge ou d'éviter la recharge sont découplées l'une de l'autre par des circuits inverseurs, diodes ou autres éléments équivalents 15 et 16.

Les caractéristiques du ou des éclateurs sont choisies pour être conducteurs et donc provoquer la décharge à un seuil de haute tension déterminé fixe correspondant à la tension nominale de charge du condensateur et à l'énergie de défibrillation voulue.

La présence de deux éclateurs permet en outre d'obtenir une sécurité contre toute surcharge du condensateur.

A titre d'exemple on peut choisir comme tension d'amorçage 2400 volts pour E1 et 2600 volts pour E2.

Le circuit selon la présente invention s'applique au cas de plusieurs électrodes extérieures, dont deux seulement 11 et 12 ont été représentées et référencées. Celles-ci sont reliées de préférence chacune entre elles par d'autres éclateurs e1, e2 permettant de les individualiser en basse tension pour, par exemple, prélever entre elles des signaux électrophysiologiques ou de les court-circuiter pour des tensions plus importantes correspondant au régime de défibrillation ou de cardioversion.

La tension d'amorçage peu élevée de ceux-ci, par exemple 90 volts, permet de garantir le court-circuit en haute tension de décharge.

L'invention a été décrite ci-dessus en détail. Il est bien entendu cependant que diverses modifications simples, adjonctions, variantes directes, substitutions par des moyens équivalents entrent dans le cadre de la présente protection.


**Revendications**

1. Circuit de charge-décharge pour le condensateur d'un défibrillateur cardiaque présentant une source de courant continu P, un circuit haute tension de charge (2), un condensateur C et un circuit de décharge (9) à travers le coeur du patient caractérisé en ce que le circuit de décharge (9) comporte outre le condensateur C, au moins un éclateur E en série avec le condensateur C, éclateur E couplé optiquement à un élément optoélectronique relié à l'entrée de remise à zéro RAZ d'un circuit de commande et d'inhibition CCI destiné à commander à partir d'un circuit décisionnel prioritaire CDP une première décharge puis à arrêter automatiquement le fonctionnement du circuit de charge pour empêcher toute recharge automatique du condensateur C après la première décharge.

2. Circuit selon la revendication 1, caractérisé en ce que le circuit de commande et d'inhibition CCI est un circuit de déclenchement à changements d'états MB.

3. Circuit selon les revendications 1 et 2, caractérisé en ce que le circuit de commande et d'inhibition est un circuit monostable M commandant le fonctionnement ou l'arrêt du circuit de charge (2) après une première décharge.

4. Circuit selon les revendications 1, 2 et 3, caractérisé en ce que la période du circuit monostable M est légèrement supérieure à la durée de charge du condensateur C.

5. Circuit selon la revendication 1, caractérisé en ce que le circuit de commande et d'inhibition CCI est une bascule B.

6. Circuit selon la revendication 1, caractérisé en ce que l'éclateur E1 est doublé par un deuxième éclateur E2 de tension d'amorçage différente couplé au même élément optoélectronique dans un boîtier (13).

7. Circuit selon la revendication 6 caractérisé en ce que le deuxième éclateur E2 est de tension d'amorçage supérieure à celle de l'éclateur E1.

8. Circuit selon les revendications 1 à 5, caractérisé en ce que le circuit de commande et d'inhibition CCI est commandé parallèlement par le circuit décisionnel prioritaire.

9. Circuit selon la revendication 6, caractérisé en ce que l'élément optoélectronique est un phototransistor PT.

10. Circuit selon les revendications précédentes, caractérisé en ce qu'il comporte plusieurs électrodes extérieures au coeur reliées entre elles par des éclateurs constituant des court-circuits pour la tension de défibrillation ou de cardioversion.

## FIG.1

## FIG.2